# EUROPEAN PATENT APPLICATION

(11) **EP 3 653 160 A1**
(43) Date of publication of application: **20.05.2020**
(21) Application number: 19209846.5
(22) Date of filing: 18.11.2019
(51) Int. Cl.: A61B 46/00, A61B 46/10

(54) **STERILE RADIOLOGICAL DRAPE**

(30) Priority: 19.11.2018 US 201862769110 P
(71) Applicant: Tidi Products, LLC, Neenah, WI 54956 (US)
(72) Inventor: Toure, Samba, Grand Blanc, MI 48439 (US); Bemman, James R., Holly, MI 48442 (US); Leece, Evalina, Chicago, IL 60647 (US)
(74) Representative: Barker Brettell LLP

(57) **Abstract**

Systems and methods for a sterile radiological drape designed to isolate the non-sterile portions of medical equipment used during surgery from the sterile zone adjacent to where medical procedures are performed are described. In one embodiment the flexible sterile drape enclosure may be incorporated into the patient drape allowing for protection and draping of both the patient and the medical equipment. In another embodiment the drape comprises a board with a hole formed therein and a sterile enclosure mounted thereto atop of a Mayo stand. This hole allows for the medical equipment to rotate into the sterile enclosure in close proximity with the sterile zone without contaminating it. The systems and methods provide advantages in that the sterile drape is easy to apply and may be moved multiple times during the surgery, which reduces the length of a medical procedure as well as the cost associated therewith.

## Description

### CROSS-REFERENCE TO RELATED APPLICATION

The present application claims priority on U.S. Provisional Patent Application Serial No. 62/769,110, filed November 19, 2018 and entitled *Sterile Radiological Drape*, the entirety of which is hereby incorporated herein by reference. The present application also claims priority on U.S. Design Patent Application Serial No. 29/649,813, filed on June 1, 2018, and entitled *Lateral C-Arm Drape*, the entirety of which is hereby incorporated herein by reference.

### BACKGROUND OF THE INVENTION

### 1. Field of the Invention

The present invention relates in general to the field of surgery. More particularly, the present invention relates to a surgical drape. Specifically, a preferred embodiment of the present invention relates to a sterile radiological drape that can be used during surgical procedures where medical equipment is moved about the operating room while maintaining sterility.

### 2. Discussion of the Related Art

Failure to maintain sterility can result in infections, which can be dangerous to a patient and expensive to the hospital. In worst case scenarios, infections can even result in death. Historically, medical procedures oftentimes utilize the draping of various sheets, sometimes called half sheets, to maintain sterile conditions about an operating room and avoid infection. A conventional half sheet is typically a plain rectangular sheet draped over the patient and down over the sides of the surgical table. For example, if a patient is having surgery on his or her arm, half sheets would typically be spread over the remaining body so as to prevent contamination.

As is known to those skilled in the art, more complex surgeries such as spinal or orthopedic surgeries require continued use of unsterilized radiological equipment, such as a C-arm, in close proximity to the patient's body and at various orientations. Thus, a previously recognized problem has been that half sheets do not prevent contamination from unsterilized equipment when it is used in close proximity to or above the patient. Needless to say, it is desirable to use the unsterilized radiological equipment without additional fear of contamination.

One unsatisfactory previously recognized approach, in an attempt to solve the problem referred to herein, involves a drape fastened to a mount near the ceiling and hanging down to the floor. A disadvantage of this previously recognized approach is that for surgeries that involve the use of a C-arm, a portion of the arm must be brought in close proximity relative to the lateral side of the patient. When this occurs, a portion of the equipment comes in contact with the surgical drape and, in fact, causes a portion of the drape below the procedure area to be brought up to, or above, waist level. This is undesirable, since it is well known that any material previously below the procedural area is considered to be out of the sterile field. Further, this previously recognized approach also has the disadvantage of cluttering the surgery room and as a result restricting the surgeon's freedom to move.

Moreover, this previously recognized solution also has the disadvantage of relatively high cost. As sales of medical device are a competitive business, a preferred solution will be seen by the end-user as being cost effective. A solution is cost effective when it is seen by the end-user as compelling when compared with other potential uses that the end-user could make of limited resources.

A number of the issues described above were largely alleviated with the introduction of the system and drape shown in U.S. Patent Nos. 8,042,549 and 8,286,637, which are incorporated herein by reference in their entirety. Still, further improvements are desired.

What is needed therefore is a surgical drape that would allow for continued use of unsterilized equipment without contaminating the sterile field. Further, what is also needed is a mechanism to use this drape without limiting the surgeon's mobility or prolonging the surgery.

### SUMMARY AND OBJECTS OF THE INVENTION

In accordance with a first aspect of the invention, the above objects are achieved by providing an apparatus comprising a sterile imaging drape for use on a piece of medical equipment to preserve a sterile field of an operating theater. The sterile imaging drape may include a flexible enclosure including a proximal end mounted to the operating table, a distal end opposite the proximal end, an outer upper portion between the proximal and distal ends, and an inner medical equipment shield portion opposite the outer upper portion. The flexible enclosure can repeatedly be moved between an expanded configuration and a collapsed configuration. In the expanded configuration, the flexible enclosure encloses at least a portion of the medical equipment. In the collapsed configuration, the outer upper portion of the flexible enclosure if folded inwardly to substantially shield the outer upper portion from external contamination. Additionally, the sterile imaging drape may include at least one reusable enclosure fastener disposed adjacent at least one of said proximal and distal ends to secure the enclosure in the collapsed configuration.

The sterile imaging drape may attach to the operating table by way of adhesive strips or fasteners and has an expandable enclosure in which the unsterilized imaging unit is contained. The inner surface of the flexible enclosure touches the medical equipment and therefore is not sterile. The outer upper surface of the flexible enclosure may be sterile sterile to avoid contaminate of a sterile zone. In one embodiment, the sterile equipment drape may be integrated into the patient drape. For instance, the flexible enclosure may have a patient drape portion and a medical equipment drape portion. The patient drape portion is configured to cover at least a portion of a patient resting on the operating table. The patient drape portion may be made of a transparent plastic material. The medical equipment drape portion has the proximal end and the distal end. The medical equipment drape portion is configured to cover the piece of medical equipment in the expanded configuration and to shield the outer upper portion in the collapsed configuration. The patient drape portion may be made of an absorbent draping material.

According to another aspect of the invention, the drape takes the form of a flexible enclosure that can be either expanded or collapsed repeatedly and deployed in less than thirty seconds. More preferably, in less that fifteen seconds. Even more preferably, in less than two seconds. In such an embodiment, when the enclosure is in the expanded position the enclosure is designed to be sufficiently sized to receive and cover at least a portion of the medical equipment, and when the enclosure is in the collapsed position the sterile portion of the enclosure is folded inwardly such that the upper sterile face is substantially shielded from external contamination, such as from external objects or the non-sterile surfaces of the enclosure itself. The enclosure may be symmetrical or asymmetrical. An electronic UV light may be used to decontaminate the sterile imaging drape. Another object of the invention is to provide a method that is predictable and reproducible, thereby decreasing variance and operating costs. Another object of the invention is to provide a method that has one or more of the characteristics discussed above but which is which is relatively simple to setup and operate using relatively low skilled workers.

In another embodiment, the drape may further include an isolation drape that extends from the flexible enclosure that separates the operating table from the medical equipment. For instance, the drape may include at least one stand that supports the isolation drape, and a pocket configured to receive a portion of the medical equipment that is formed in a bottom surface of the isolation drape. More specifically, a first vertical stand and a second vertical stand may support the isolation drape, and the flexible enclosure may be the pocket such that a portion of the medical equipment may extend beneath the isolation drape into the pocket. In this configuration, the pocket can be moved from the expanded configuration to the collapsed configuration.

In still another embodiment, the enclosure may have an integrated tenting feature, such as a manipulable strip, which would allow the drape to be suspended in a given position. For instance, the strip may be located at the distal end, which enables a collapsible integrated tenting feature to be formed. Alternatively still, the strip may be an aluminum strip that allows the flexible enclosure to be held at table height while the medical device is rotated in and out of the tent or sterile pocket. The strips are malleable and allow for the expanding of the enclosure. The tenting feature may be collapsed to allow for disposing the drape within the sterile field through adhesive strips of fasteners.

In still yet another embodiment, the sterile drape comprises an upward facing cone-shaped device to prevent dust, microparticles, or debris from the drape to contact the patient.

In still yet another embodiment, the distal end of the sterile drape is mounted to the operating table by way of adhesive strips or fasteners and the proximal end comprising the sterile enclosure may be collapsed using an accordion-style sidewall that allows the sidewall and the outer upper portion to fold and be disposed to substantially shield the outer upper portion from external contamination and thus preserve the sterility of the sterile zone. The collapsed accordion style enclosure may be secured through use of fasteners or adhesive strips.

According to another aspect of the invention, the sterile drape may also have a board that is connected to the flexible enclosure and mounted adjacent to the operating table. The board may further include an opening formed therein, where at least a portion of the medical equipment is movable through the opening and into the flexible enclosure to shield the outer upper portion. The sterile drape may be clear to allow for observation of the imaging tube interaction with the patient. Alternatively, the board and/or sterile enclosure may be mounted to a Mayo stand with an opening in the bottom through which the medical equipment enters the sterile enclosure.

In another embodiment, the sterile drape may be stored in a hoop position atop the hole in the board or Mayo stand. As the medical equipment is rotated into the sterile field it passes through the hoop and expands the sterile drape. As the medical equipment is rotated out of the sterile field the sterile drape collapses back into the hoop.

In still another embodiment, the sterile drape may be in the shape of a domed bag and/or mounted to a board and form a sterile tube for the medical equipment to pass through in a telescoping manner. The board may be secured under the operating table pad and the sterile tube may extend from below the table to table-height.

The invention is also directed to a sterile drape system comprising a plurality of flexible sheet enclosures, wherein one of the plurality of flexible sheets is removed when the medical equipment is moved about the operating theater. The drape may be further coated with an anti-microbial substance to kill bacteria.

In one embodiment of the sterile drape system, the flexible sheet enclosures are mounted on top of one another around the medical equipment. Once the medical equipment is moved near the sterile field, the outermost sheet is removed and disposed of so that a new sterile sheet is on the outer surface the medical equipment as it is rotated into the sterile field.

In another embodiment, the sterile drape system is stored on a roll which is attached to the operating table. The sterile drape being drawn from the roll to cover the medical equipment and then torn from the roll and disposed of. Each time the medical equipment is rotated into the sterile field the medical equipment is covered with a new sterile drape from the roll. The roll may be spring loaded.

Although the above discussion has focused exclusively on the design and features of the device itself, the current invention is also directed to a method of draping an operating room during a surgical procedure requiring imaging that uses the unique features of the inventive drape. For instance, the flexible enclosure described above may be affixed to an operating table. Thereafter, the flexible enclosure may be moved to the expanded configuration where the flexible enclosure encloses at least a portion of the medical equipment. Once the medical equipment has been used, the flexible enclosure may be moved to the collapsed configuration where the outer upper portion of the flexible enclosure is folded inwardly to substantially shield the outer upper portion from external contamination. The flexible enclosure can then be secured in the collapsed configuration using at least one reusable enclosure fasteners. The method may also include the steps of securing a patient drape portion of the flexible enclosure over a patient resting on the operating table and securing a medical equipment drape portion adjacent to an end of the operating table where a portion of the medical equipment is moved. Additionally, the method may include installing at least one stand adjacent to the operating table, affixing an isolation drape having a pocket formed therein to the at least one stand, and moving a portion of the medical equipment into the pocket. Further still, the method may include the steps of moving the flexible enclosure to the expanded configuration, after which at least one manipulable strip formed in the distal end may be manipulated to support the flexible enclosure. Also, the method may include the steps of installing a board having an opening formed therein with the flexible enclosure extending therefrom adjacent to the operating table, moving a portion of the medical equipment relative to the opening, moving the portion of the medical equipment through the opening, and moving the portion of the medical equipment into the flexible enclosure.

According to another aspect of the invention, another method is described. In a first step, the sterile drape is to be spread on the floor of the operating room to begin creating a sterile barrier. In the second step, the sterile drape may then be spread underneath the operating table, thus forming a separate sterile field under the operating table. This additional sterile field could eliminate the need for systems that require disposing of multiple drapes each time the medical equipment enters the sterile field by converting the majority of the operating room into a sterile field. Thus, reducing costs and time required for surgery.

These, and other aspects and objects of the present invention will be better appreciated and understood when considered in conjunction with the following description and the accompanying drawings. It should be understood, however, that the following description, while indicating preferred embodiments of the present invention, is given by way of illustration and not of limitation. Many changes and modifications may be made within the scope of the present invention without departing from the spirit thereof, and the invention includes all such modifications.

### BRIEF DESCRIPTION OF THE DRAWINGS

A clear conception of the advantages and features constituting the present invention, and of the construction and operation of typical mechanisms provided with the present invention, will become more readily apparent by referring to the exemplary, and therefore non-limiting, embodiments illustrated in the drawings accompanying and forming a part of this specification, wherein like reference numerals designate the same elements in the several views, and in which:
FIG. 1 illustrates a perspective view of a sterile drape incorporated into a patient drape with a piece of medical equipment expanding a flexible sterile enclosure, in accordance with the present invention.
FIG. 2 illustrates a perspective view of a sterile drape suspended from two stands to form an isolation wall with the lower portion comprising a flexible sterile enclosure, where the sterile drape is in an initial position, in accordance with the present invention.
FIG. 3 illustrates a perspective view of the sterile drape suspended from two stands of FIG. 2, where the sterile drape is in an expanded position, in accordance with the present invention.
FIG. 4 illustrates a perspective view of a sterile drape with an integrated tenting feature consisting of aluminum strips expanding the flexible sterile enclosure, in accordance with the present invention.
FIG. 5 illustrates a low cost sterile drape for use to with a movable piece of medical equipment.
FIG. 6 illustrates a perspective view of a sterile drape which disposes into the sterile field in an accordion folding manner
FIGS. 7A and 7B illustrates perspective views of a sterile drape forming an enclosure mounted to a board and secured under the operating table pad, in accordance with the present invention.
FIG. 8 illustrates a perspective view of a sterile drape forming an enclosure mounted to a Mayo stand, in accordance with the present invention.
FIG. 9 illustrates a perspective view of the sterile drape mounted to the Mayo stand for FIG. 8, where medical equipment is moved into the enclosure, in accordance with the present invention.
FIG. 10 illustrates a perspective view of a sterile drape stored in a hoop on a Mayo stand, in accordance with the present invention.
FIG. 11A-E illustrate a side view of a sterile drape forming a sterile tube extending from below the operating table and mounted to a board which is secured under the operating table pad, in accordance with the present invention.
FIGS. 12 illustrates a side view of a drape system wherein a plurality of flexible sheet enclosures is mounted on top of one another and secured around the medical equipment, in accordance with the present invention.
FIG. 13 Illustrates a perspective view of the drape system wherein a plurality of flexible sheet enclosures is stored on a roll, in accordance with the present invention.
FIG. 14 illustrates the method using a sterile drape, in accordance with the present invention.

In describing the preferred embodiment of the invention which is illustrated in the drawings, specific terminology will be resorted to for the sake of clarity. However, it is not intended that the invention be limited to the specific terms so selected and it is to be understood that each specific term includes all technical equivalents which operate in a similar manner to accomplish a similar purpose. For example, the word connected, attached, or terms similar thereto are often used. They are not limited to direct connection but include connection through other elements where such connection is recognized as being equivalent by those skilled in the art.

### DESCRIPTION OF PREFERRED EMBODIMENTS

The present invention and the various features and advantageous details thereof are explained more fully with reference to the non-limiting embodiments described in detail in the following description.

As shown in the figures, the present invention is directed to a sterile radiological drape 20 for use during surgical procedures. This sterile radiological drape 20 helps to maintain sterility about an operating theater 26 while various medical equipment 22 is moved about an operating table 32. For instance, the drape 20 can be used with imaging devices, which enables easy transition between surgery and radiological imaging during medical procedures. The sterile radiological drape 20 allows for multiple uses of unsterilized medical equipment 22 without contaminating the sterile field 24. Preferably, the sterile radiological drape 20 can be easily and affordably manufactured, while also being of high quality and durability. Various non-limiting embodiments of drapes that fulfill these requirements will be further described below. Any of the features associated with any of these embodiments may be combinable with any of the other embodiments shown herein.

In many of the embodiments described below, the sterile radiological drape 20 may include a proximal end 29 and distal end 31 with a flexible sterile enclosure 28 therebetween. Furthermore, the drape 20 may include an inner medical equipment shielding portion 34 and an outer upper portion 36. The inner medical equipment shielding portion 34 need not be sterile, and repeatedly can come into contact with the medical equipment 22. The sterile drape 20 which may attach to the operating table 32 by way of adhesive strips or fasteners (not shown).

Additionally, the drape 20 may include reusable enclosure fasteners 38 on both the proximal end 29 and distal end 31 that allow the drape 20 to move back and forth between expanded and collapsed positions. More specifically, when in the expanded position the drape 20 is designed to be sufficiently sized to receive and cover at least a portion of the unsterilized medical equipment 22, such as an unsterilized imaging unit. When this occurs, the equipment 22 may be housed within the enclosure 28 and abut against the inner medical equipment shielding portion 34. When the drape 20 is returned to the collapsed position, the proximal end 29 and the distal end 31 are brought together, and the outer upper portion 36 of the enclosure is folded inwardly such that the outer upper portion 36 is substantially shielded from external contamination, such as from external objects or the non-sterile surfaces of the enclosure itself. The drape 20 may be an accordion-style drape that is mounted to the edge of the operating table 32.

Preferably, the drape 20 can be either expanded or collapsed repeatedly in an expedited manner to improve efficiency. For instance, in one embodiment, the drape 20 can be expanded or collapsed in less than thirty seconds. More preferably, the drape 20 can be expanded or collapsed in less that fifteen seconds. Even more preferably, the drape can be expanded or collapsed in less than two seconds.

In many of the illustrated embodiments, the drape 20 is symmetrical. However, the drape 20 could similarly be asymmetrical. For instance, the drape 20 may be asymmetrical in order to adequately and securely cover a specific piece of medical equipment. Furthermore, while the figures show a number of different drapes having a number of different dimensions and shapes, the specific shape and size of the drape 20 could vary significantly depending on a number of different factors, such as the medical equipment 22 it is designed to cover, the size of the operating theater 26, the specific medical procedure being performed, and the like.

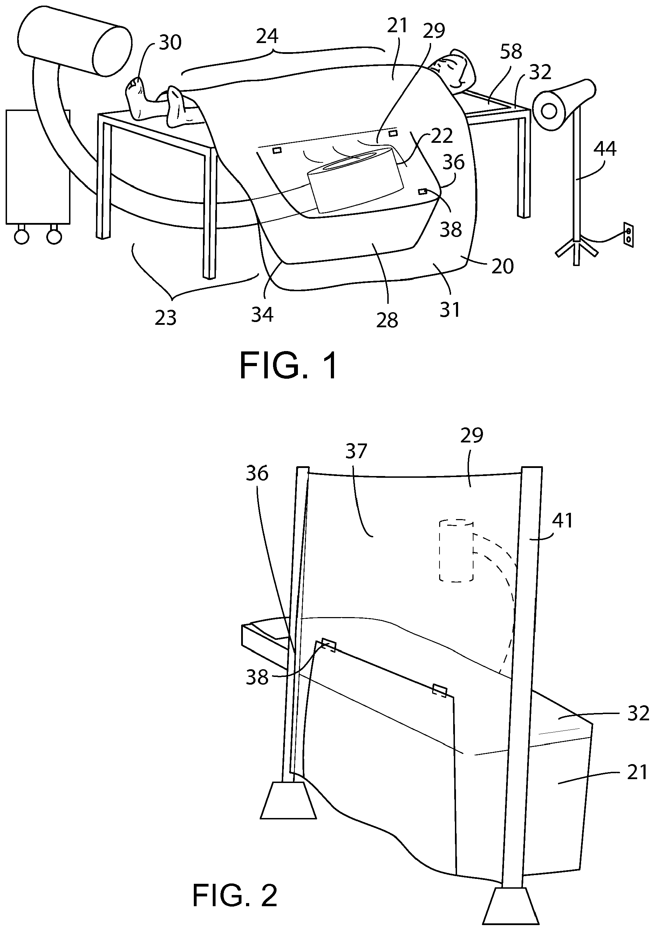

Turning now to the embodiment shown in FIG. 1, the sterile radiological drape 20 is integrated with a patient drape 21. In doing so, a single drape can easily be installed onto the patient 30 and operating table 32 before the medical procedure commences. The integrated sterile radiological drape 20 and patient drape 21 could be manufactured together or manufactured separately and later secured to one another. Additionally, the integrated sterile radiological drape 20 and patient drape 21 can be folded in such a way as to simplify the process of unwrapping and installing it to the patient 30 and operating table 32 while maintaining sterility about the operating theater 26. Alternatively, the patient 30 may be draped after the possible contamination of an adjacent piece of medical equipment 22 to avoid exposing the patient to the possible contamination. The sterile radiological drape 20 portion could be made of the same material as the patient drape 21, or it could be made of different materials. For instance, all portions could be made of an absorbent blue draping material. Alternatively, the sterile radiological drape 20 portion could be made of a transparent plastic material and the patient drape could be made of an absorbent blue draping material. The material may also be coated with an anti-microbial substance.

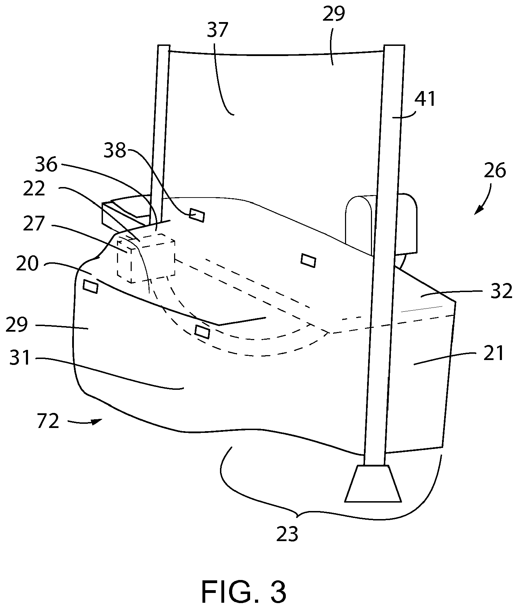

Moving on to FIGS. 2 and 3, the drape 20 could take the form of an isolation drape. More specifically, as shown in initially in FIG. 2, the drape 20 may include a portion that serves as a vertical isolation drape 37 that separates the operating table 32 and the patient 30 from the exterior of the isolation drape 37, and a pocket 27 formed in the bottom of the isolation drape 37. The isolation drape 37 can extend vertically between two stands 41, which help to form an isolation drape. The pocket 27 may be configured to accept various medical equipment 22 when the medical equipment 22 is moved past the isolation drape 37. For instance, the drape 20 may be used in connection with an X-ray tube. In this embodiment, the lower half of the drape 20 has the pocket 27 into which the medical equipment 22 is raised when in use. More specifically, the pocket 27 can be manipulated when the medical equipment 22 is moved outside of the isolation drape 37. When the pocket 27 is manipulated, it can be opened such that the medical equipment 22 is located therebeneath to maintain sterility about the operating theater 26. Additionally, the drape 20 and/or pocket 27 may be made of, or include, absorbent materials to help absorb any liquids that enter the drape 20 or the pocket 27. Further still, a drain (not shown) may be formed in the center to control the location with which liquids exit the drape 20 and/or pocket 27.

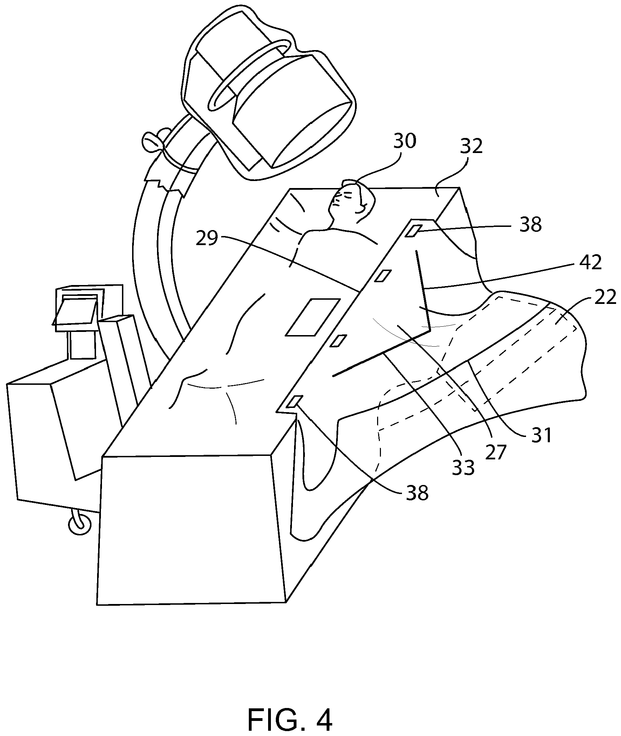

Turning next to FIG. 4, another embodiment is shown in which the drape 20 may include various components to facilitate various tenting effects, including an integrated tenting feature 33. In the illustrated embodiment, the integrated tenting feature 33 of the drape 20 includes at least one bendable strip 42 is incorporated with a portion of the drape 20. For instance, the bendable strip 42 may be a malleable aluminum strip that can easily be bended, but thereafter holds its shape. Of course, the bendable strip 42 could similarly be made of other malleable materials. This would allow a user to adjust the strip 42 in a specific location such that the drape 20 will retain a position shape or configuration. For instance, a user could position the strip 42 such that the drape 20 would be suspended at table height and for the medical equipment 22 to be rotated in and out of the pocket 27. As shown, the bendable strip 42 is located on the distal end 31 of the drape 20, such that the bendable strip 42 can be positioned when the drape 20 is in an expanded configuration. More specifically, when the drape 20 is moved to the expanded position, the bendable strip 42 can be bent to ensure that the drape 20 remains secured around the medical equipment 22 until the medical equipment 22 is returned to its original position, in which case the bendable strip 42 can be moved away from the medical equipment 22. The tenting feature may be collapsed, which in turn would allow the drape 20 to be collapsed as described above.

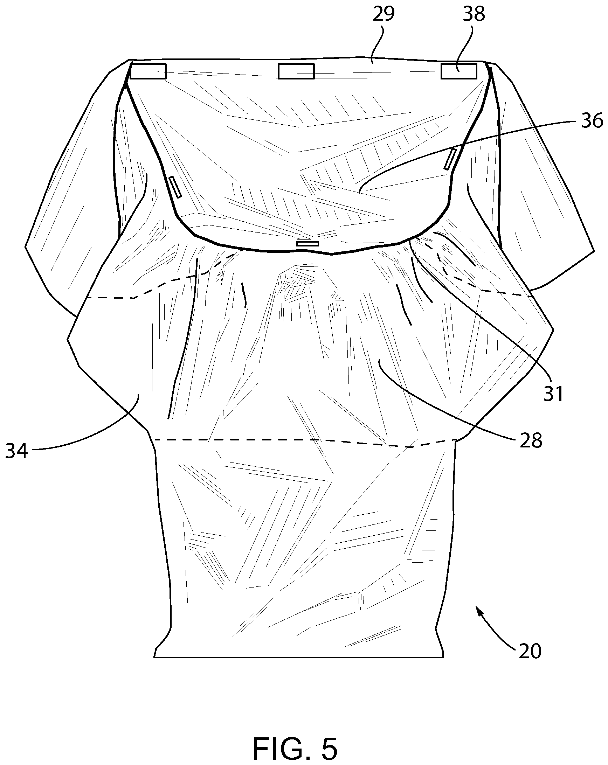

In yet another embodiment, a drape 20 may be desired that can be affordably manufactured. For instance, one such drape 20 is shown in U.S. Design Pat. App. No. 29/649,813, which is incorporated herein by reference in its entirety and attached hereto. An image of the affordable, low-cost drape 20 is shown in FIG. 5, which shows the drape 20, including the proximal end 29, the distal end 31, the inner medical equipment shield portion 34, and the outer upper portion 36, as well as the reusable enclosure fasteners 38, in isolation. Such a drape 20 may provide 5-sided protection to ensure sterility is maintained about the patient 30 and the operating table 32.

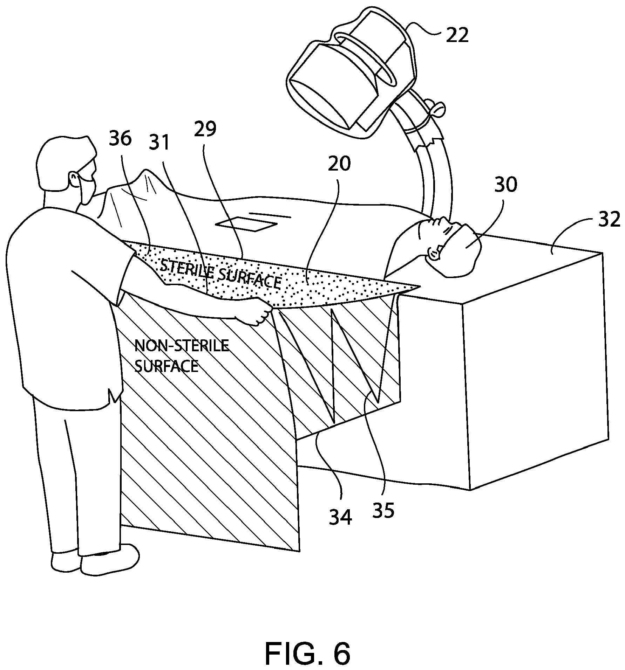

Looking now at FIG. 6, another embodiment is shown in which the drape 20 is shown having many of the same component parts listed above, with the addition of features that allow the drape 20 to fold in an accordion-style manner 35. As shown, the accordion folding style 35 allows the outer upper portion 36 to collapse in on itself so as to not contact the inner medical equipment shielding portion 34, thus preserving sterility to the sterile field. The accordion folding style 35 of the drape 20 helps to ensure that the sidewalls 39 fold inwardly to maintain sterility outside of the drape 20 while components located inside the drape 20, including the medical equipment 22, may not be sterile. This allows the medical equipment 22 to be safely moved about the operating theater 26 without subjecting the patient to non-sterile conditions. Again, the drape 20 may be repeatedly moved from an expanded configuration as shown in FIG. 6 to a collapsed position when the medical equipment 22 is moved away from the operating table 32.

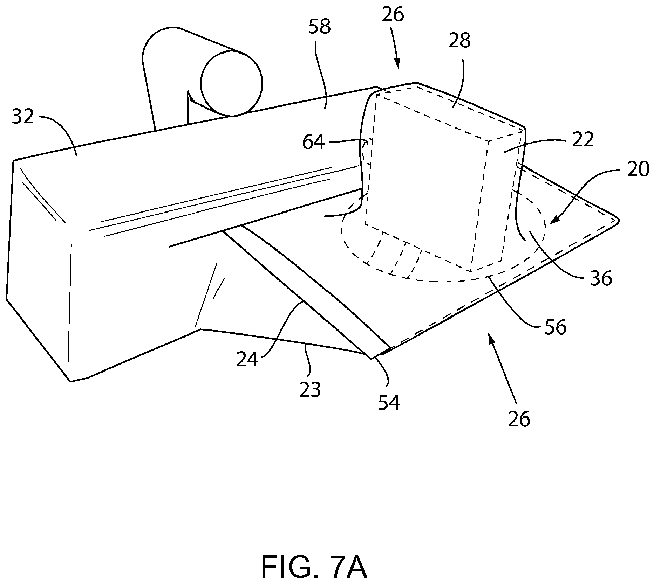

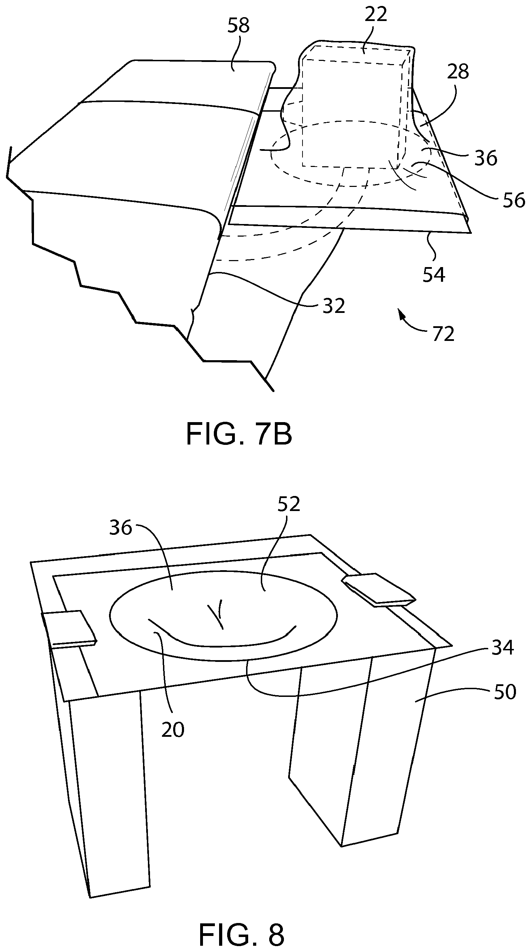

Other components could also be used with the drape 20 to help maintain sterility of about the operating theater 26 while the medical equipment 22 is moved. For instance, as shown in FIGS. 7A and 7B, the sterile drape 20 may also form an enclosure 28 which may be mounted to a board 54. These figures show the drape 20 when the medical equipment 22 is moved within the enclosure 28. The board 54 may be made of a thin, durable material to provide added rigidity to the drape 20 system. The board 54 may be secured under the operating table pad 58 and may have a bottom opening 56 or hole through which the medical equipment 22 can pass through to enter the sterile enclosure 28. While the illustrated embodiments include a substantially square-shaped board 54 with a circular opening 56 formed therein, both the board 54 and the opening 56 may be in different configurations depending on a number of factors, including the size and shape of the medical equipment 22, the desired movements of the medical equipment 22, the medical procedures being performed, the size of the operating table and theater 26, 32, and the like. As shown, the board 54 will remain in place relative to the operating table 32, and the medical equipment 22, such as an imaging tube 64, can be repeatedly be moved through and out of the opening 52 into and out of the enclosure 28. The sterile drape 20 may be clear to allow for observation of the imaging tube 64 as it interacts with the patient 30.

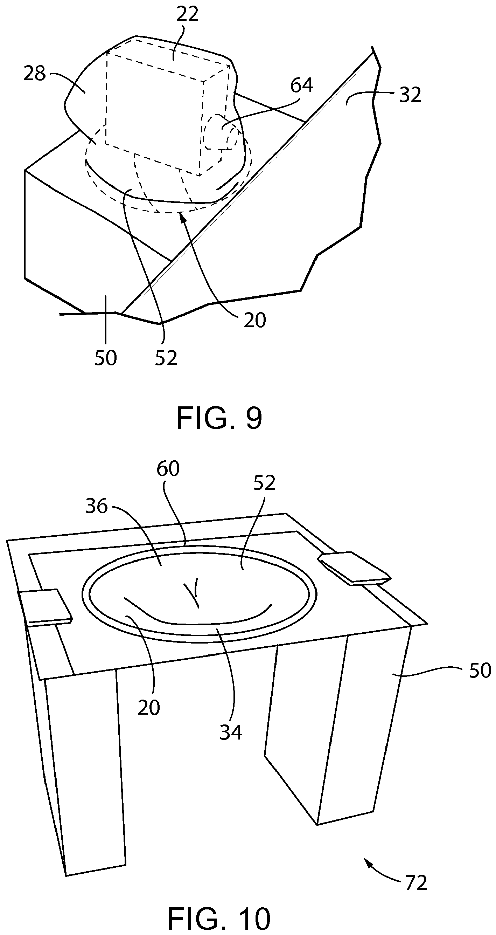

Turning next to FIGS. 8 and 9, another similar embodiment is provided, that also includes a board 54 with an opening 56 extending therethrough. However, the board is mounted to a Mayo stand 50. For instance, FIG. 8 shows the drape 20 system in an initial position before it is installed adjacent to the operating table 32 and before the medical equipment 22 is moved into the enclosure 28. First, the drape 20 system is moved adjacent to the operating table 32. Thereafter, when the medical equipment 22 is moved from a first position to a second position, it can be moved into the enclosure 28 as shown in FIG. 9. When this occurs, the medical equipment 22 moves through the bottom opening or hole 56 into the sterile enclosure 28. Again, the medical equipment 22 can be repeated moved into and out of the enclosure 28 without compromising sterility of the drape system.

In yet another embodiment shown in FIG. 10, the sterile drape 20 may be stored in a hoop 60 position atop the hole 52, 56 in the board 54 or Mayo stand 50. Alternatively still, the hoop 60 may be used with the drape 20 in the absence of a board 54 or Mayo stand 50. Again, the hoop 60 provides structural rigidity to the drape 20 system. As the medical equipment 22 is rotated into the sterile field 24 it passes through the hoop 60 and expands the sterile drape 20. As the medical equipment 22 is rotated out of the sterile field 24 the sterile drape 20 collapses back into the hoop 60. Of course, the hoop 60 may be mounted to the table 32 or any other device in the operating theater 26.

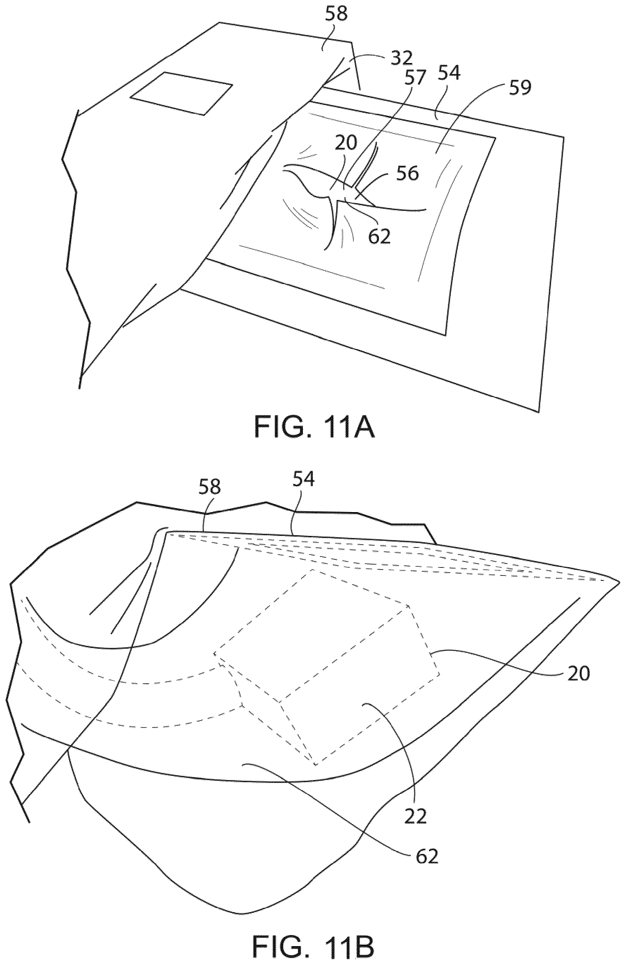

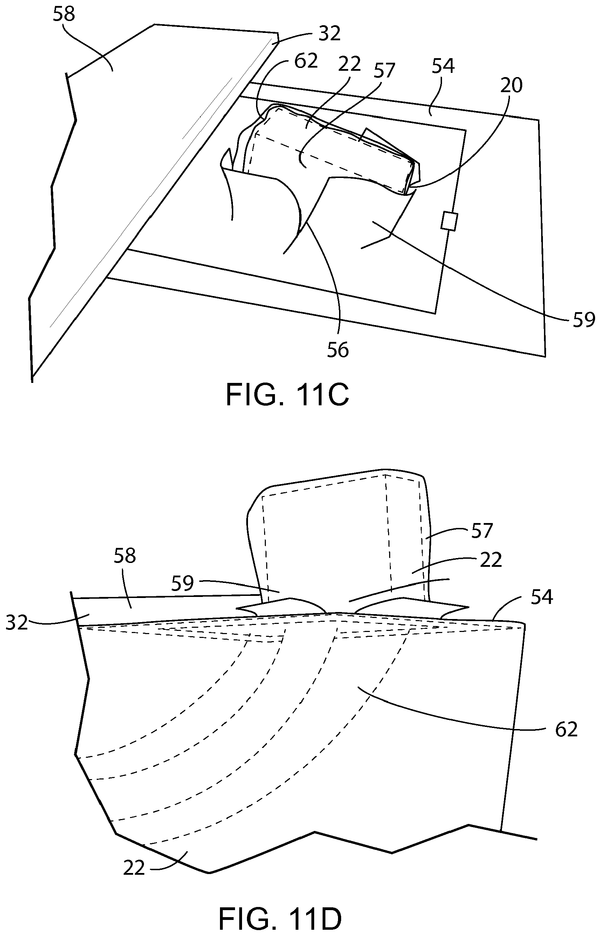

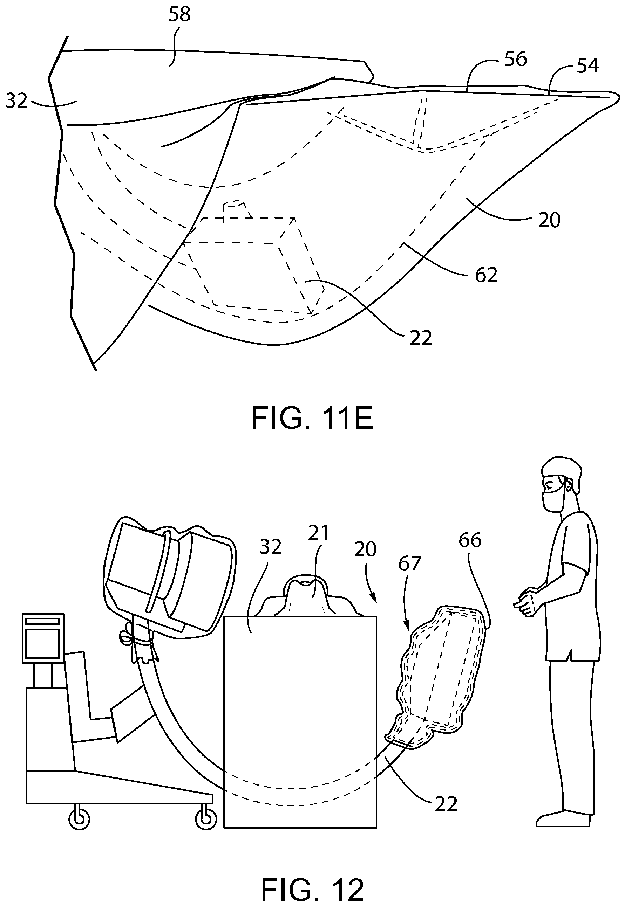

In still another similar embodiment shown in FIGS. 11A-11E, the sterile drape 20 may again include to a board 54 with a sterile tube 62 extending therebeneath for the medical equipment 22 to pass through in a telescoping manner. More specifically, a sterile tube 62 may extend beneath the operating table 32 to provide a clear path for the medical equipment 22 to follow when it is moved about the operating theater 26, and more specifically, beneath the table 32. The board 54 may be secured under the operating table pad 58 and the sterile tube 62 may extend from below the table to table-height. The opening 56 in the board 54 may be covered with a transparent layer 57 and a blue poly layer 59. Again, this would allow for the piece of medical equipment 22 to move about the operating theater 26 while maintaining sterility about the patient 30 and operating table 32. For instance, FIG. 11A shows the drape 20 in an initial position before the medical equipment 22 is moved, and where the blue poly layer 59 is intact. FIG. 11B shows the medical equipment 22 being moved along the sterile tube 62 towards the board 54. FIG. 11C shows the medical equipment 22 moving upwardly past the board, in which the blue poly layer 59 is separated, and the transparent layer 57 surrounds the medical equipment 22 and moves upwardly with the transparent layer 57 to ensure the medical equipment 22 is fully sounded, and thus not capable of compromising sterility about the operating theater 26. FIG. 11D also shows a side view of the drape 20 system once the medical equipment 22 has moved through the board 54. Finally, FIG. 11E shows the medical equipment 22 after it is returned through the sterile tube 62 beneath the operating table 32.

In other embodiments, a drape system 67 may be provided having include multiple sheets of material. For instance, the sterile drape system 67 may include a plurality of flexible sheet enclosures 66 that are disposable. As seen in FIG. 12, a plurality of flexible sheet enclosures 66 are used to cover a piece of medical equipment 22 such as an x-ray tube. Any time the piece of medical equipment 22 is returned to an opposite side of the operating table 32, the flexible sheet enclosure 66 that contacted the piece of medical equipment 22 can be removed and disposed of so that a new sheet is on the outer surface of the medical equipment 22 once it is rotated back relative to the operating table 32. For instance, the flexible sheet enclosures 66 may be dome bags or sheets. Additionally, the flexible enclosure 66 may be ripped or teared apart from the remaining flexible enclosure 66. To further facilitate the separation of flexible enclosures from the system 67, various perforations may be provided.

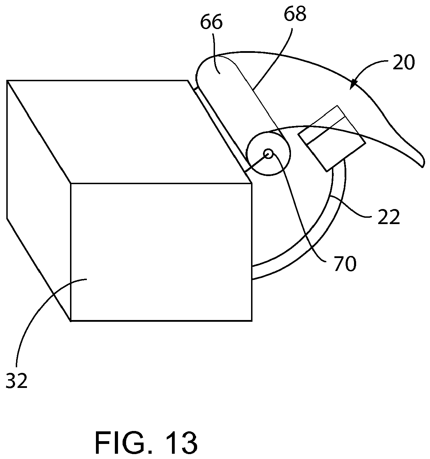

On a similar note, FIG. 13 provides drape system with a dispenser roll 68 on which multiple flexible sheet enclosures 66 may be mounted. As shown, the dispenser roll 68 may be attached to the operating table 32. The flexible sheet enclosure 66 may be drawn from the roll 68 to cover the medical equipment 22 and then torn from the roll 68 and disposed of once the medical equipment 22 is moved. Each time the medical equipment 22 is rotated into the sterile field 24 the medical equipment 22 is covered with a new sterile drape 20 from the roll 68. Additionally, the roll 68 may include a spring 70 such that the roll 68 is spring-loaded for ease of dispensing. The system 67 may also include various sterile towels (not shown).

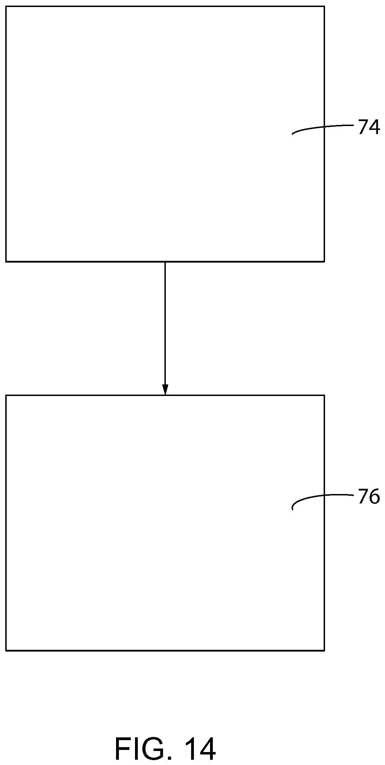

Although the above discussion has focused exclusively on the design and features of the device itself, the current invention is also directed to a method of draping an operating theater/room 26 during a surgical procedure requiring imaging that uses the unique features of the inventive drape. For instance, as shown in FIG. 14, in a first step 74, the sterile drape 20 is to be spread on the floor of the operating theater 26 to begin creating a sterile barrier. In the second step 76, the sterile drape 20 may then be spread underneath the operating table 32, thus forming a separate sterile field under the operating table 32. This additional sterile field 24 could eliminate the need for systems that require disposing of multiple drapes each time the medical equipment 22 enters the sterile field 24 by converting the majority of the operating theater into a sterile field 24. Thus, costs and time required for surgery can be reduced.

Additionally, various manufacturing and packaging processes may be implemented to improve the efficiency of creating the sterile imaging drape 20. For instance, an electronic UV light (not shown) may be used to decontaminate the drape 20. Similarly, a specific method of manufacture can be implemented that is predictable and reproducible, thereby decreasing variance and operating costs. Further still, a method may be implemented ease the simplicity of setup, manufacture, and packaging of the drape 20. On a related note, the drape 20 may be packaged in a way to help ensure that sterility is maintained while the drape is unfolded and applied to the operating table 32.

Although the best mode contemplated by the inventors of carrying out the present invention is disclosed above, practice of the present invention is not limited thereto. It will be manifest that various additions, modifications and rearrangements of the features of the present invention may be made without deviating from the spirit and scope of the underlying inventive concept. For example, any of the specific aspects of any of the described embodiments could similarly be used with any of the other embodiments. Furthermore, while specific materials have been described, it should be known that any materials could used to create any of the described drapes. For instance, materials may be chosen based on any number of criteria, including costs, availability, and various sterility properties. Moreover, as described above, the individual components need not be formed in the disclosed shapes, or assembled in the disclosed configuration, but could be provided in virtually any shape, and assembled in virtually any configuration. Further, any of the components can be manufactured with one another or be separately manufactured and later assembled. Furthermore, all the disclosed features of each disclosed embodiment can be combined with, or substituted for, the disclosed features of every other disclosed embodiment except where such features are mutually exclusive.

It is intended that the appended claims cover all such additions, modifications and rearrangements. Expedient embodiments of the present invention are differentiated by the appended claims.

## Claims

1. A sterile imaging drape for use on a piece of medical equipment to preserve a sterile field of an operating theater comprising:
a flexible enclosure attached to an operating table including:
a proximal end mounted to the operating table;
a distal end, opposite the proximal end;
an outer upper portion between the proximal and distal ends; and
an inner medical equipment shielding portion, opposite the outer upper portion wherein the flexible enclosure can repeatedly be moved between;
an expanded configuration where the flexible enclosure encloses at least a portion of the piece of medical equipment; and
a collapsed configuration where the outer upper portion of the flexible enclosure is folded inwardly to substantially shield the outer upper portion from external contamination; and
at least one reusable enclosure fastener disposed adjacent at least one of said proximal and distal ends to secure the enclosure in the collapsed configuration.

2. The sterile imaging drape of claim 1, wherein the flexible enclosure further comprises:
a patient drape portion configured to cover at least a portion of a patient resting on the operating table; and
a medical equipment drape portion having the proximal end and the distal end configured to cover the piece of medical equipment in the expanded configuration and configured to shield the outer upper portion in the collapsed configuration,
optionally wherein the medical equipment drape portion is made of a transparent plastic material and the patient drape portion is made of an absorbent draping material.

3. The sterile imaging drape of claim 1 or claim 2, further comprising an isolation drape extending from the flexible enclosure that separates the operating table from the piece of medical equipment.

4. The sterile imaging drape of claim 3, further comprising:
at least one stand supporting the isolation drape; and
a pocket formed in a bottom surface of the isolation drape;
wherein the pocket is configured to receive a portion of the piece of medical equipment.

5. The sterile imaging drape of claim 4, wherein the at least one stand comprises a first vertical stand and a second vertical stand;
wherein the isolation drape is vertical isolation drape supported by the first vertical stand and the second vertical stand;
wherein the flexible enclosure is the pocket; and
wherein a portion of the piece of medical equipment extends beneath the isolation drape into the pocket,
optionally wherein the distal end of the pocket can be moved:
to the expanded configuration to accept the portion of the piece of medical equipment; and
to the collapsed configuration when the portion of the piece of medical equipment is removed from the pocket.

6. The sterile imaging drape of any preceding claim, further comprising at least one manipulable strip near the distal end forming a collapsible integrated tenting feature, optionally wherein the at least one manipulable strip comprises an aluminum strip that is bendable to hold the flexible enclosure at table height.

7. The sterile imaging drape of any preceding claim, further comprising adhesive mounted adjacent to the distal end configured to mount the flexible enclosure to an operating table,.the sterile imaging drape optionally further comprising an accordion folding sidewall allowing for the sidewall and the outer upper portion to fold and be disposed to substantially shield the outer upper portion from external contamination.

8. The sterile imaging drape of any preceding claim, further comprising:
a board connected to the flexible enclosure and mounted adjacent to the operating table; and
an opening formed in the board;
wherein at least a portion of the piece of medical equipment is moveable through the opening into the flexible enclosure to shield the outer upper portion, optionally wherein the board is mounted to a Mayo stand.

9. The sterile imaging drape of claim 8, further comprising a reinforced hoop surrounding the opening;
wherein the flexible enclosure extends from the hoop in the expanded configuration; and
wherein the flexible enclosure folds into the hoop in the collapsed configuration.

10. The sterile imaging drape of claim 8 or claim 9, further comprising a sterile tube extending beneath the operating table;
wherein the portion of the piece of medical equipment travels through the sterile tube, through the opening, and into the flexible enclosure.

11. A method of draping an operating room comprising the steps of:
affixing a flexible enclosure to an operating table, the flexible enclosure including:
a proximal end mounted to the operating table;
a distal end, opposite the proximal end;
an outer upper portion between the proximal and distal end; and
an inner medical equipment shielding portion, opposite the outer upper portion;
moving the flexible enclosure to an expanded configuration where the flexible enclosure encloses at least a portion of the piece of medical equipment;
moving the flexible enclosure to a collapsed configuration where the outer upper portion of the flexible enclosure is folded inwardly to substantially shield the outer upper portion from external contamination; and
securing the flexible enclosure in the collapsed configuration using at least one reusable enclosure fasteners.

12. The method of claim 11, further comprising the steps of:
securing a patient drape portion of the flexible enclosure over a patient resting on the operating table; and
securing a medical equipment drape portion adjacent to an end of the operating table where a portion of the piece of medical equipment is moved.

13. The method of claim 11 or claim 12, further comprising the steps of:
installing at least one stand adjacent to the operating table;
affixing an isolation drape having a pocket formed therein to the at least one stand; and
moving a portion of piece of medical equipment into the pocket.

14. The method of any of claims 11 to 12, further comprising the steps of:
moving the flexible enclosure to the expanded configuration; and
manipulating at least one manipulable strip formed in the distal end to support the flexible enclosure.

15. The method of any of claims 11 to 14, further comprising the steps of:
installing a board having an opening formed therein with the flexible enclosure extending therefrom adjacent to the operating table;
moving a portion of the piece of medical equipment relative to the operating table;
moving the portion of the piece of medical equipment through the opening; and
moving the portion of the piece of medical equipment into the flexible enclosure.
